# EUROPEAN PATENT APPLICATION

(11) **EP 3 578 110 A1**
(43) Date of publication of application: **11.12.2019**
(21) Application number: 18176455.6
(22) Date of filing: 07.06.2018
(51) Int. Cl.: A61B 10/00

(54) **METHOD AND APPARATUS FOR SAMPLING AND IMAGING**

(71) Applicant: Nokia Technologies Oy, 02610 Espoo (FI)
(72) Inventor: Müller, Kiti, 00430 Helsinki (FI); Rajala, Satu, 36200 Kangasala (FI)
(74) Representative: Espatent Oy

(57) **Abstract**

An apparatus, comprising a first chamber; a second chamber; imaging means positioned in the first chamber and configured to image a target area associated with a surface; actuator means configured to bring a sampling element to a sampling position at a first end of the second chamber; wherein the sampling element is configured to sample a substance from the surface when positioned at the sampling position; and control means.

## Description

### TECHNICAL FIELD

The present application generally relates to a method and apparatus for sampling and imaging. In particular, but not exclusively, the present application relates to a method and apparatus for fluid sampling an imaging.

### BACKGROUND

This section illustrates useful background information without admission of any technique described herein representative of the state of the art.

Human body fluids are versatile indicators of health and sickness. The quality and representativeness of a sample of a body fluid is often dependent on the location as well as method of sampling. Furthermore, the characteristics of the sampling location might play a role in determining the usefulness of the sample. Furthermore, in repeated sampling, it may be useful to take samples from a consistent location.

For example human saliva and the characteristics of human tongue can be used for various purposes. Commonly, the tongue is examined visually and saliva samples are taken either by spitting into a test tube or by absorbent test element placed on or swept over the tongue.

### SUMMARY

Various aspects of examples of the invention are set out in the claims.

According to a first example aspect of the present invention, there is provided an apparatus comprising:
a first chamber;
a second chamber;
imaging means positioned in the first chamber and configured to image a target area associated with a surface;
actuator means configured to bring a sampling element to a sampling position at a first end of the second chamber; wherein the sampling element is configured to sample a substance from the surface when positioned at the sampling position;
and
control means.

The apparatus may further comprise means for illuminating the target area.

The apparatus may further comprise means for measuring a quantity relating to the target area to be imaged.

The actuator means may comprise a piston for causing an underpressure inside the second chamber for maintaining the sampling element at the sampling position.

The apparatus may further comprise storage means configured to store one or more sampling elements, wherein the actuator means may be configured to release a sampling element from the storing means to bring it to the sampling position.

The sampling element may comprise an absorbent material.

The sampling element may comprise an aperture and the imaging means may be configured to image the target area through the aperture.

The sampling element may comprise a functional portion.

The apparatus may comprise means configured to cause:
the imaging means to image the target area of the surface; and
the actuation means to bring the sampling element to the sampling position at
the first end of the second chamber.

The means for imaging may comprise an imaging unit comprising elements such as a charge coupled device, CCD, imaging sensor, a complementary metal oxide semiconductor, CMOS, imaging sensor, a photometric stereo imaging system, or a further digital imaging system.

The actuator means may comprise an actuator unit comprising elements such as a piston configured to cause a change of the pressure inside the outer chamber.

The control means may comprise a control unit comprising elements such as a processor and a memory.

The means for illuminating the target area may comprise an illumination unit comprising elements such as a light emitting diode, LED, or an array thereof.

According to a second example aspect of the present invention, there is provided a method comprising:
imaging a target area associated with a surface with imaging means positioned in a first chamber of an apparatus; and
bringing a sampling element into a sampling position at a first end of a second chamber of the apparatus; wherein the sampling element is configured to sample a substance from the surface when positioned at the sampling position.

The method may further comprise illuminating the target area.

The method may further comprise measuring a quantity relating to the target area.

Bringing the sampling element to the sampling position may comprise causing an underpressure inside the second chamber for maintaining the sampling element at the sampling position.

Bringing the sampling element to the sampling position may comprise releasing a sampling element from a storage means.

Imaging the target area may comprise imaging the sampling location through an aperture in the sampling element.

According to a third example aspect of the present invention, there is provided a computer program comprising computer executable program code configured to execute any method of the second example aspect.

The computer program may be stored in a computer readable memory medium.

Any foregoing memory medium may comprise a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

According to a fourth example aspect of the present invention, there is provided an apparatus comprising a memory and a processor that are configured to cause the apparatus to perform the method of the first example aspect.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1A shows a schematic side view of an apparatus according to an example embodiment;
Fig. 1B shows a further schematic side view of an apparatus according to an example embodiment;
Fig. 1C shows a schematic plan view of an apparatus according to an example embodiment;
Fig. 2 shows a schematic side view of an apparatus according to a further example embodiment;
Fig. 3 shows a block diagram of a part of an apparatus according to an example embodiment; and
Fig. 4 shows a flow chart of a method according to an example embodiment.

### DETAILED DESCRIPTON OF THE DRAWINGS

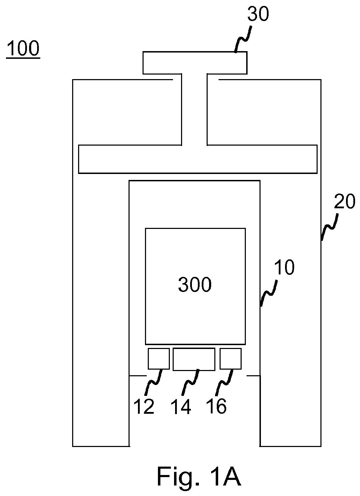

An example embodiment of the present invention and its potential advantages are understood by referring to Figs. 1A through 4 of the drawings. In this document, like reference signs denote like parts or steps.

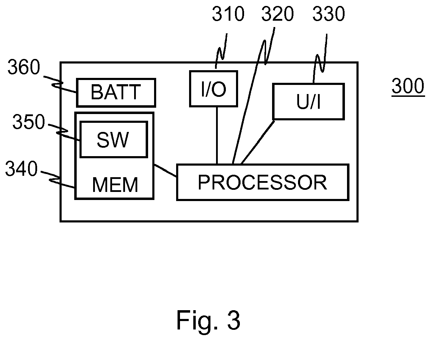

Fig. 1A shows a schematic side view of an apparatus 100 according to an example embodiment. The apparatus 100 comprises, in an example embodiment, a first chamber 10 and a second chamber 20. In an example embodiment, the first chamber 10 comprises an inner chamber and the second chamber 20 comprises an outer chamber. In an example embodiment, the first chamber 10 and the second chamber 20 form a pen-like enclosure for the apparatus 100. In an example embodiment, the inner chamber 10 comprises a control means 300 configured to control the elements and functions of the apparatus. In an example embodiment, the control means 300 comprises a control unit comprising elements such as a processor and a memory (not shown in Fig 1A) as will be described hereinafter in more detail with reference to Fig. 3.

It is to be noted that in a further example embodiment, the control means 300 is comprised in a different part of the apparatus 100, or is comprised in a further apparatus outside the apparatus 100 in which case the control means 300 is configured to be connected with and communicate with the elements of the apparatus 100 either with a wired connection or wirelessly using a suitable communications protocol.

The first chamber 10 of the apparatus 100 further comprises, in an example embodiment, illumination means configured to illuminate a target. The first end of the first chamber 10 is open to the surrounding and the illumination means 12 is configured to direct electromagnetic radiation, for example visible, infrared or ultraviolet light on the target, for example on a surface on which the apparatus is placed. In an example embodiment, the target comprises an area associated with the surface, such as an area of the surface, a protrusion on the surface, an area located on the surface or an anomaly on the surface. In an example embodiment, the illumination means comprises an illumination unit comprising elements such as a light emitting diode, LED, or an array thereof. In an example embodiment, the illumination means 12 is configured to be controlled by the control means 300 in order to control the illumination. In an example embodiment, controlling the illumination comprises controlling the intensity, the hue, the wavelength and/or direction of illumination.

The first chamber 10 of the apparatus 100 further comprises imaging means 14. In an example embodiment, the imaging means 14 is configured to image a target, such as a surface on which or at distance from which the apparatus is placed. In an example embodiment, the target comprises an area associated with the surface, such as an area of the surface, a protrusion on the surface, an area located on the surface or an anomaly on the surface. In an example embodiment, the imaging means comprise an imaging unit comprising elements such as a charge coupled device, CCD, imaging sensor, a complementary metal oxide semiconductor, CMOS, imaging sensor, a photometric stereo imaging system, or a further digital imaging system. In an example embodiment, the imaging means 14 is configured to be controlled by the control element 300.

The first chamber 10 of the apparatus 100 further comprises sensor means 16. In an example embodiment, the sensor means 16 is configured to measure and/or detect a quantity relating to a target, such as a surface on which or at distance from which the apparatus is placed. In an example embodiment, the sensor means 16 comprise a sensor unit comprising elements such as a temperature sensor, such as an infrared temperature sensor. In a further example embodiment, the sensor means 16 comprise several sensors configured to measure the same quantity or different quantities. In an example embodiment, the sensor means 16 is configured to be controlled by the control element 300.

The second chamber 20 of the apparatus 100 comprises, in an example embodiment, an actuator means 30. The actuator means 30 comprise in an example embodiment an actuator unit comprising elements such as a piston configured to cause a change of the pressure inside the outer chamber 20. In an example embodiment, pulling the piston upwards causes an underpressure inside the second chamber 20 so that suction is created at the first end of the second chamber which is open to the surroundings. The actuator means 30 is in an embodiment positioned at a second end of the second chamber, opposite to the first end of the second chamber 20. In an example embodiment, pressing the piston downwards releases the underpressure and/or causes an overpressure inside the second chamber 20. Fig. 1A shows the piston being pressed down.

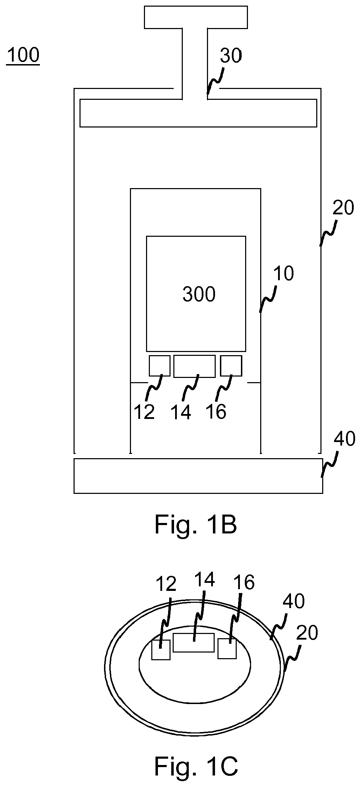

Fig. 1B shows a further schematic side view of an apparatus 100 according to an example embodiment. Fig.1B shows the elements of the apparatus 100 as hereinbefore described with reference to Fig. 1A. In Fig. 1B, the actuator means 30, i.e. the piston has been pulled upwards in order to cause an underpressure inside the second chamber 20 in order to bring, i.e. suck, a sampling element 40 onto the first end of the second chamber 20. In an embodiment, the actuator means 30 comprises means for measuring the pressure needed to hold the sampling element 40 in place at the first end of the second chamber in order to assess the amount of fluid sampled. The sampling element comprises, in an example embodiment, an element of material configured to absorb the fluid to be sampled, such as an absorbent paper, absorbent pad or an absorbent strip. In further example embodiment, the sampling element 40 comprises instead of or in addition to absorbent characteristics a functional portion, such as a portion comprising a reagent, i.e. in an example embodiment the sampling element comprises a rapid test element. A rapid test element may be a test element configured to change color in response to the presence of a reagent.

Fig. 1C shows a schematic plan view of an apparatus according to an example embodiment. The plan view shows an example of the apparatus in an example orientation on a horizontal plane, such that the illumination means 12, the imaging means 14, and sensor means 16 are visible. It should be noted that use of the apparatus is not limited to the example orientation. Fig. 1C shows the illumination means 12, the imaging means 14 and the sensor means 16 visible from the middle of the sampling element 40 maintained by suction at the first end of the second chamber 20. In an example embodiment, the sampling element 40 has a ringlike form so that the line of sight from the end part of the first chamber 10 comprising the illumination means 12, the imaging means 14 and sensor means 16 is not hindered. The second chamber, in such an example embodiment corresponds to the shape of the sampling element 40, i.e. the second chamber 20 surrounds the first chamber 10 and has a ringlike cross section in the plane shown in Fig. 1C.

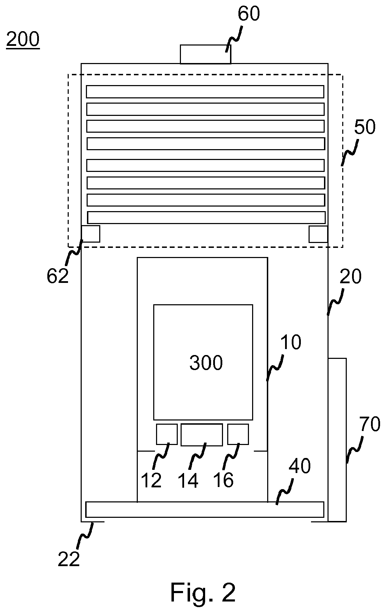

Fig. 2 shows a schematic side view of an apparatus 200 according to a further example embodiment. The apparatus 200 according to an example embodiment, similarly to the apparatus 100, comprises a first chamber 10, a second chamber 20, a control means 300, an illumination means 12, an imaging means 14, and a sensor means 16, the structure and functionality of which corresponds to that described with reference to Figs. 1A-1C.

The apparatus 200 according to an example embodiment further comprises a storage means 50 configured for storing and dispensing sampling elements 40.. In a further example embodiment, the storage means 50 is formed as a further chamber and attached to the second chamber 20. In an example embodiment, the storage means is configured to store sampling elements 40, for example stacked in a pile on top of one another.

The apparatus 200 according to an example element further comprises an actuator means 60 configured to bring a sampling element 40 from the storage element 50 to the first end of the second chamber 20. In an example embodiment, the actuator means 60 comprises a button, the pressing of which is configured to release a sampling element 40 from the storage means so that it is brought to the first end of the second chamber 20.

In an example embodiment, the actuator means 60, directly or through the control means 300, is configured to actuate a holder element 62 at the first end of the storage means 50 thus causing a sampling element 40 to drop towards the first part of the second chamber 20. In an example embodiment, the first end of the second chamber 20 comprises a stopper element 22 configured to hold the sampling element 40 in a sampling position. In an example embodiment, the stopper element 22 comprises means, such as a strain gauge, for measuring the force caused by the sampling element in place thereon at the first end of the second chamber in order to assess the amount of fluid sampled. In an example embodiment, the actuator means 60 is configured to, directly or through the control means 300, to release the stopper element in such a way that the sampling element 40 is released from the first end of the second chamber 20.

In an example embodiment, the apparatus 200 further comprises a reagent providing means 70 configured to provide, for example spray or inject, a reagent on the sampling element 40 on the first end of the second chamber 20. It is to be noted that also the apparatus 100 explained hereinbefore with respect to Figs. 1A-1C in an example embodiment comprises a reagent providing means 70.

In a further example embodiment, independent of the method of bringing the sampling element 40 to the first end of the second chamber 20, the sampling element 40 has a shape different from a ring, for example rectangular or oval form with an aperture in the middle. In an example embodiment, the form of the cross section of the at least the second chamber 20 corresponds to the form of the sampling element 40. In a further example embodiment, independent of the method of bringing the sampling element 40 to the first end of the second chamber 20, the second chamber 20 is located next to the first chamber 10 instead of therearound. In such an example embodiment, the sampling element 40 need not have an aperture in the middle as the illumination means 12, the imaging means 14 and the sensor means 16 are located next to the sampling element 40 instead of in the middle thereof.

Fig. 3 shows a block diagram of the control means 300 according to an example embodiment of the invention. The control means 300 comprises a memory 340 including computer program code 350. In an example embodiment, the memory 340 comprises a memory medium comprising a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

The control means 300 further comprises a processor 320 for controlling the operation of an apparatus using the computer program code 350, a communication unit 310 for communicating with other apparatuses and/or with the elements of the apparatus 100,200, and in an example embodiment a user interface unit 330. In an example embodiment, the user interface unit comprises, on the outer surface of the apparatus 100,200 elements for controlling the functions of the apparatus 100,200.

In an example embodiment, the communication unit 310 is configured to provide communication with an external apparatus such as a smartphone, a smartwatch, a tablet computer, a laptop computer, a desktop computer, laboratory equipment, or a cloud service. The communication unit 310 comprises, for example, a local area network (LAN) port, a wireless local area network (WLAN) unit, Bluetooth unit, cellular data communication unit, or satellite data communication unit.

The processor 320 comprises, for example, any one or more of: a master control unit (MCU), a microprocessor, a digital signal processor (DSP), an application specific integrated circuit (ASIC), a field programmable gate array, and a microcontroller. In a further example embodiment, the control unit 300, or a part of it is comprised in an external apparatus or in a cloud service.

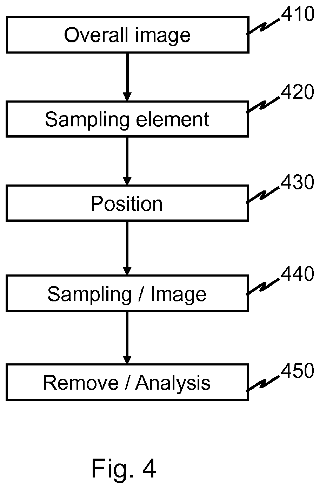

Fig. 4 shows a flow chart of a method according to an example embodiment. At step 410 an overall image of the general area, for example a human tongue, on which the sampling and examination is to be carried out is taken using the imaging means 14 of the apparatus 100,200. From the overall image, a sampling location is selected either automatically or manually. In a further example embodiment, the overall image has been previously taken, using the imaging means 14 or with another imaging apparatus and the overall imaging need not be repeated, for example in case of repetitive sampling in long term monitoring of a single sample location.

At step 420 the sampling element 40 is brought to sampling position at the first end of the second chamber 20. In an example embodiment, the sampling element 40 is brought into the sampling position by actuating the actuator means 30 in such a way as to create an underpressure to the second chamber 20 and bringing the sampling element 40 thereon by sucking it from an external storage of sampling elements. In a further example embodiment, the sampling element 40 is brought into the sampling position by actuating the actuating means 60 in order to release a sampling element 40 from the storage means 50.

At step 430 the apparatus 100,200 and accordingly the sampling element 40 is set at the location to be sampled and examined, for example on the surface of the human tongue or skin. In an example embodiment, the position to which the apparatus 100,200 is set is the position determined at step 410, but the apparatus can also be set on a randomly selected position if no need has arisen for a certain sampling position. As the apparatus 100,200 is set on the location, the sampling element comes into contact with the fluid to be sampled, for example saliva or sweat.

At step 440, the actual sampling and imaging takes place. The sampling element 40 absorbs, or otherwise attaches to itself a sample of the fluid to be sampled. Concurrently therewith the location being sampled is imaged. Hereto it is noted that the imaging, in an embodiment, is carried out before the sampling is finished, i.e. sampling in an embodiment lasts longer, or imaging is carried out even before the sampling has started, i.e. while approaching the location. In a further example embodiment, the imaging is carried out after the sampling is already finished.

The imaging in an example embodiment comprises illuminating the target to be imaged with the illuminating means 12 from at least one direction with at least one type of electromagnetic radiation and imaging with the imaging means 14. In a further example embodiment, no illumination is used, or several types of illuminations from different directions are used. Furthermore, in an example embodiment, a quantity, for example temperature, relating to the sampling location is measured during sampling and imaging using the sensor means 16.

At step 450 after the sampling and imaging has been carried out, the apparatus is removed from the sampling location and subsequently the sampling element 40 is released from the apparatus 100,200. In an example embodiment, prior to releasing, the amount of fluid sampled is estimated by an element of the actuator 30 or of the stopper element 22 as hereinbefore described. Furthermore, in an example embodiment, during one or several of the steps 420 to 450, a reagent or further chemical is provided on the sample element 40 using the reagent providing means 70 of the apparatus 100,200.

The sampling element 40 with the fluid sampled is released from the apparatus 100,200 for storage, further analysis or reading of the result in case a rapid test element or reagent from the reagent providing means was used. The steps after the release of the sampling element 40 depend on the situation, i.e. from the tests to be carried, delay in testing and further processing or transport required. In an example embodiment, if the sampling element 40 comprises a rapid test element or a reagent for a rapid test was provided, the sampling element 40 is imaged with the imaging means 14 during or after the sampling.

In an example embodiment, the images taken and/or the result of any test carried out on the sample taken is analyzed and processed, for example at the control unit 300 or in a further device connected therewith, such as a smartphone, and the results are then are shown to the user and/or stored for further use and monitoring purposes.

Without in any way limiting the scope, interpretation, or application of the claims appearing below, a technical effect of one or more of the example embodiments disclosed herein is the provision of objective measurement and documentation of the characteristics of the sampling area. The sampling area may be for example the human tongue, mouth cavity, skin or a wound. The characteristics may comprise presence of bacteria. Another technical effect of one or more of the example embodiments disclosed herein is the provision of an easy way to gather a body fluid or sweat, such as saliva from a certain location also for long term monitoring. Another technical effect of one or more of the example embodiments disclosed herein is the integration of sampling and determination of the characteristics of the sampling location into a single apparatus. Another technical effect of one or more of the example embodiments disclosed herein is an improved quality and comparability of sampling and examination results.

Embodiments of the present invention may be implemented in software, hardware, application logic or a combination of software, hardware and application logic. The software, application logic and/or hardware may reside on the control element 300. If desired, part of the software, application logic and/or hardware may reside on the control element 300, part of the software, application logic and/or hardware may reside on an external device, and/or part of the software, application logic and/or hardware may reside on a cloud service. In an example embodiment, the application logic, software or an instruction set is maintained on any one of various conventional computer-readable media. In the context of this document, a "computer-readable medium" may be any non-transitory media or means that can contain, store, communicate, propagate or transport the instructions for use by or in connection with an instruction execution system, apparatus, or device, such as a computer, with one example of a computer described and depicted in Fig. 3. A computer-readable medium may comprise a computer-readable storage medium that may be any media or means that can contain or store the instructions for use by or in connection with an instruction execution system, apparatus, or device, such as a computer.

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications which may be made without departing from the scope of the present invention as defined in the appended claims.

## Claims

1. An apparatus comprising:
a first chamber;
a second chamber;
imaging means positioned in the first chamber and configured to image a target area associated with a surface;
actuator means configured to bring a sampling element to a sampling position at a first end of the second chamber; wherein the sampling element is configured to sample a substance from the surface when positioned at the sampling position; and
control means.

2. The apparatus of claim 1, further comprising means for illuminating the target area.

3. The apparatus of claim 1 or 2, further comprising means for measuring a quantity relating to the target area to be imaged.

4. The apparatus of any preceding claim, wherein the actuator means comprises a piston for causing an underpressure inside the second chamber for maintaining the sampling element at the sampling position.

5. The apparatus of any of the claims 1 to 3, further comprising storage means configured to store one or more sampling elements, wherein the actuator means is configured to release a sampling element from the storing means to bring it to the sampling position.

6. The apparatus of any preceding claim, wherein the sampling element comprises an absorbent material.

7. The apparatus of any preceding claim, wherein the sampling element comprises an aperture and the imaging means is configured to image the target area through the aperture.

8. The apparatus of any preceding claim, wherein the sampling element comprises a functional portion.

9. The apparatus of any preceding claim, comprising means configured to cause:
the imaging means to image the target area of the surface; and
the actuation means to bring the sampling element to the sampling position at the first end of the second chamber.

10. A method comprising:
imaging a target area associated with a surface with imaging means positioned in a first chamber of an apparatus; and
bringing a sampling element into a sampling position at a first end of a second chamber of the apparatus; wherein the sampling element is configured to sample a substance from the surface when positioned at the sampling position.

11. The method of claim 10, further comprising illuminating the target area.

12. The method of claim 10 or 11, further comprising measuring a quantity relating to the target area.

13. The method of any of claims 10 to 12, wherein bringing the sampling element to the sampling position comprises causing an underpressure inside the second chamber for maintaining the sampling element at the sampling position.

14. The method of any of claims 10 to 12, wherein bringing the sampling element to the sampling position comprises releasing a sampling element from a storage means.

15. The method of any of claims 10 to 14, wherein imaging the target area comprises imaging the sampling location through an aperture in the sampling element.
